# EUROPEAN PATENT APPLICATION

(11) **EP 2 016 888 A1**
(43) Date of publication of application: **21.01.2009**
(21) Application number: 08008897.4
(22) Date of filing: 14.10.2005
(51) Int. Cl.: A61B 3/107, A61B 3/15, A61B 3/18, A61B 17/42, A61B 3/00, A61B 3/11, A61B 3/14

(54) **Corneal topography apparatus with further integrated devices**

(62) Divisional of application: 05805155.8
(71) Applicant: HESP Technology S.r.l., 52027 San Giovanni Valdarno (IT)
(72) Inventor: Pasquini, Paolo, 52024 Loro Ciuffenna (AR) (IT); Foggi, Alessandro, 52027 San Giovanni Valdarno (AR) (IT)
(74) Representative: Celestino, Marco

(57) **Abstract**

An apparatus for corneal topographic analysis, determining the precise curvature of a cornea and the position of the optical axis giving a map of such curvature to an operator, and also for pupillometric analysis, determining the pupil contour and the relative centre both in steady and dynamic conditions during transients of contraction and dilation, and for a real analysis by fluoresceine by testing a contact lens fitting on a cornea. The apparatus comprises a measuring head (6) having a Placido disc (2) backlighted with a light source (31), suitable for causing the pupil to contract. Coaxial to the disc (2) a videocamera (10) is mounted, comprising an optical unit (11) for focusing on a shot image. The measuring head (6) comprises a plurality of infrared LEDs, for example respectively four LEDs (14,15,16,17), suitable for lighting the eye in a way suitable not to disturb the aperture of the pupil and two visible light LEDs (18,19) arranged on the Placido disc (2) suitable for causing the contraction of the pupil.

## Description

### Field of the invention

The present invention relates to the field of ophthalmology and, in particular, it relates to a corneal topography apparatus with further integrated devices suitable for carrying out additional diagnostics.

### Background of the invention

As well known, in the field of ophthalmology, examinations of the geometry of the cornea and of the pupil are very important. Such examinations are necessary, in particular, in case of some pathologies, as well as to assist an eye surgical operation for correcting morphological defects of the cornea, for example in case of laser surgical operation, for reducing the defects of myopia. In this case, in fact, not only it is relevant to have extremely precise data relatively to the shape of the cornea, but it is also necessary to have very precise data on the shape of the pupil, not only in the condition of maximum and minimum aperture but also during transients, during which the centre of the pupil moves. In this way risks are avoided that, under certain conditions, after the operation part of the pupil extends beyond the area of operation, creating sight defects at the edge of the area of operation due to imperfections.

In the field of contactology it is relevant to know precisely curvature of the cornea, in order to simulate the behaviour of a contact lens located on the cornea and then to determine, among a set of lenses, the best fitting lens. By pupillometry, moreover, indispensable data can be obtained to know the visual quality, through the cornea and at different apertures of the pupil, in the presence of a contact lens that changes the visual characteristics of an eye. Furthermore, for evaluating the correct fitting of a contact lens on the cornea, it is useful to carry out a real analysis by fluoresceine on the cornea with the contact lens applied thereon. Normally this analysis is carried out with a slit lamp which uses a blue light and a yellow filter.

Among the existing devices, documents W00232298 and EP1371322A1 describe an ophthalmic measuring device comprising a corneal topography apparatus having a pupillometer integrated in it. In both cases the apparatus comprises a video camera with a path coaxial to a Placido disc, having a fixation visible light LED that can align the eye to the videocamera. Furthermore, it comprises an infrared LED, which is located on the same path of the camera, and that by means of its reflection guides a doctor to align the videocamera with the optical axis of the eye of the patient, and to determine, by suitable calculations, the position of the optical axis on the image. The apparatus comprises, furthermore, a visible neon light located behind the cone, used for bringing the pupil to a minimum dilation for making the measurements. The light reflected by the surface of the cornea and previously projected by the Placido disc, instead, allows to determine the corneal topography. Both known devices comprise also a plurality of infrared light LEDs, arranged laterally on the Placido disc, for lighting the eye with grazing light and for allowing measurements of the pupil.

EP1371322A1 has the drawback that a dynamic measurement of the shape of the pupil is not provided during a transient corresponding to pupil contraction (passage from scotopic to photopic condition), and only an analysis at pupil dilation (passage from photopic condition to scotopic) is allowed. In fact, in EP1371322A1 the visible light source that causes the pupil to contract is located behind the Placido disc. Then, the portion of visible light that reaches the surface of the cornea is split into a plurality of concentric rings, corresponding to transparent rings of the Placido disc; this light, reflected by the cornea, is detected by the videocamera and overlaps to the image of the pupil, disturbing the detection of the pupil contour, which cannot be obtained accurately.

Another drawback of EP1371322A1 is that this device detects the optical axis of the eye reading the position of a small lighted area created by the infrared light of the fixation LED as reflected by the cornea, obtaining the position of the optical axis as average on the pixels read by the sensor of the videocamera. In this case, the measure could result inaccurate, because miscalculations can derive from an evaluation based on a very limited number of pixels sensed by the videocamera.

### Summary of the invention

It is a feature of the present invention to provide a corneal topography apparatus capable of detecting images and films of the real behaviour of a contact lens on a cornea carrying out a real analysis by fluoresceine.

It is, furthermore, a feature of the invention to provide a device capable of carrying out, in a single workstation, an analysis of the surface of the cornea by the topography, an accurate exam on the pupil dynamics in different lighting conditions during the contraction and dilation phases, and an actual fitting status of a contact lens on a cornea.

It is then a feature of the present invention to provide a measuring system for ophthalmology comprising a corneal topography apparatus and a pupillometer integrated in it that have improved features with respect to the prior art.

It is another feature of the invention to provide a pupillometer integrated in a corneal topography apparatus capable of measuring with efficiency the centre of the pupil and the variation of its position during a dynamic measurement from the scotopic to the photopic condition (pupil contraction) and vice-versa (pupil dilation).

A further feature of the invention is to provide a pupillometer integrated in a corneal topography apparatus capable of measuring the shape of the pupil and its variation during a dynamic measurement.

Another feature of the invention is to provide a pupillometer integrated in a corneal topography apparatus capable of measuring with maximum precision the exact curvature of the cornea.

According to the invention, a diagnostic apparatus in the field of ophthalmology comprises:
- a videocamera;
- corneal topography means associated to said videocamera, for carrying out a corneal topography of an eye,
- testing means, associated to said videocamera and to said means for carrying out a corneal topography, for testing operatively the correct fitting of said eye with a contact lens on said cornea.

Advantageously, said apparatus comprises:
- a Placido disc having a plurality of concentric alternated transparent and not transparent rings;
- means for backlighting said disc.

In particular, said testing means comprises:
- light means for exciting an amount of a contrast means, in particular, fluoresceine, operatively put between the cornea and the inner surface of a contact lens applied on said cornea, said videocamera being suitable for measuring the light emitted by said contrast means when excited;
- means for analysing said light emitted by said contrast means and detected by said videocamera.

In particular, said light means for exciting said contrast means are selected from the group comprised of:
- at least one light source, in particular a LED, which emits a bright spectrum suitable for exciting said contrast means;
- at least one light source, in particular a LED, which emits visible light, associated to a filter having a pass band containing the spectrum of excitation of said contrast means.

Advantageously, said at least one source that emits a bright spectrum suitable for exciting said contrast means is located in a position selected from the group comprised of:
- laterally along the external boundary of said Placido disc;
- back behind the Placido disc;
- applied in a respective hole made on said Placido disc;
- in a position suitable for being projected towards the eye along the fixation axis, in particular, being incident on a beam splitter.

Advantageously, said at least one source that emits a bright spectrum suitable for exciting said fluoresceine has a colour having at least one peak transmission value with wavelength suitable for exciting said fluoresceine.

In a particular exemplary embodiment, said apparatus comprises a filter selected from the group comprised of:
- a fixed filter arranged on the optical path between the eye and said videocamera, said filter being suitable for cutting the wavelength lower than a predetermined value less than the minimum wavelength of interest for analysis;
- a releasable selective filter suitable for causing only the wavelength to pass that is set in the spectrum of transmission of said contrast means, where in particular said releasable filter causes only the wavelength to pass that corresponds to the spectrum of transmission of excited fluoresceine.

In particular, said fixed filter is obtained on said beam splitter arranged along the optical path between the eye of the patient and the videocamera, said beam splitter being capable of cutting the wavelength lower than a predetermined value less than the minimum wavelength of interest for analysis.

Advantageously, said apparatus comprises means for determining a simulation of the position of a contact lens on the cornea, comprising:
- means for loading from a first memory the coordinates of a lens;
- means for loading from a second memory the coordinates of the cornea calculated in a previous step;
- means for subtracting the coordinates of the lens and of the cornea, obtaining data that describe a gap between lens and cornea;
- means for tracing a map of said gap on a monitor.

Means are provided, in particular, for comparing the results of the application of said testing means relative to a previous analysis with the results of the application of said testing means relative to a successive analysis.

In a preferred exemplary embodiment, said apparatus comprises:
- first light means for lighting the pupil with infrared light in a step of measuring the pupil contour;
- second light means for determining the optical axis, said second light means being coincident with said first light means;
- light means for exciting an amount of fluoresceine operatively put between the cornea and the inner surface of a contact lens applied on said cornea.

This way, the above described apparatus is capable to carry out a dynamic measurement of the geometry of the pupil, of the geometry of the cornea and of testing operatively the correct fitting of a contact lens on a cornea, this apparatus integrating the above described functions.

By the apparatus according to the invention a simulation can be made of the position of a contact lens on the cornea, by using:
- means for loading from a first memory the coordinates of a lens;
- means for loading from a second memory the coordinates of the cornea calculated in a previous step;
- means for subtracting the coordinates of the lens and of the cornea, obtaining data that describe a gap between lens and cornea;
- means for tracing a map of said gap on a monitor.

Means can also be provided for comparing the results of the application of said testing means relative to a previous analysis with the results of the application of said testing means relative to a successive analysis.

Adventageously, said first and second light means coincide with each other.

Advantageously, said apparatus comprises fixation light means, suitable to align the eye with said videocamera, wherein said videocamera has an optical path, said fixation light means comprising a visible fixation light source arranged laterally with respect to said optical path, said fixation light being incident on a semitransparent lamina, of beam splitter type, arranged inclined with respect to said fixation light and to said optical path, in order to direct said fixation light along said optical path. This way, said light is partially reflected by the beam splitter and crosses the centre of the Placido disc in order to be visible by the patient only when the eye is aligned with the videocamera same. Then, to align the eye the patient has simply to look at the above described fixation light source.

In particular, said means for backlighting said disc comprises a light source selected from the group comprised of:
- at least one fluorescent light source, in particular neon light;
- at least one infrared light source;
- at least one visible LED light source;
- a plurality of visible light LEDs, said LEDs being arranged on a circumference that is concentric to said disc and is located behind said disc.

In particular, said first light means and said second light means that are coincident to each other comprise a plurality of infrared light sources, in particular LEDs, located eccentrically on the surface of said Placido disc according to a geometry such that the barycentre of their images reflected on the eye coincides with said optical axis.

In a preferred exemplary embodiment, said plurality of infrared light sources comprises a number of light sources arranged at a short distance from the centre of said Placido disc respectively at the vertices of a regular triangle, square, pentagon, hexagon, etc., concentric with said disc. In particular, said plurality of infrared light sources is arranged on a first opaque ring of said Placido disc.

Advantageously, said apparatus comprises means for lighting the eye with visible light for causing the contraction of the pupil. This way, in fact, by turning on said means for lighting it is possible to carry out a measurement of the variation of the shape of the pupil in photopic condition starting from a scotopic condition where the eye is not yet hit by visible light.

In particular, said means for lighting the eye with visible light comprises at least one visible light source, in particular a LED, located eccentrically on the surface of said Placido disc and oriented towards the eye, in a direction that is not grazing with respect to the eye.

In one preferred exemplary embodiment, said means for lighting the eye with visible light comprises two light sources, located at a short distance from the centre of said Placido disc and diametrically opposite from each other with respect to said centre.

In particular, the side surface and/or a rear face of said infrared light sources and of said visible light source(s) are coated with opaque material, in order not to provide any backlighting to said disc.

In particular, said infrared light sources and/or said visible light source(s) are put in corresponding holes made through the above described Placido disc. This way, the rear part of the disc is not lighted avoiding that on the eye the rings of the disc reflect. In fact, the light delivered by said source is sent only in a desired direction, and not along or behind the Placido disc, and thus avoiding noise in the measurements.

The apparatus according to the invention in addition to carrying out a topographic analysis for detecting the precise curvature of a cornea and the position of the optical axis giving a map of such curvature to an operator, it can carry out a real analysis by fluoresceine for testing a contact lens fitting on a cornea, in particular responsive to said curvature. Moreover, it can carry out a pupillometric analysis detecting the pupil contour and the relative centre both in steady and dynamic conditions during transients of contraction (passage from scotopic to photopic condition) and dilation (passage from photopic condition to scotopic). Furthermore

The images can be obtained even as a film. Such images can be transferred in real time to a computer connected to the apparatus according to the invention. By carrying out two or three different analysis types on a same machine as above described, it is possible to save all the images obtained on a same computer and to analyse them or to compare them with other images present on a data-base.

### Brief description of the drawings

Further characteristics and the advantages of the apparatus for ophthalmology, according to the invention, will be made clearer with the following description of an exemplary embodiment thereof, exemplifying but not limitative, with reference to the attached drawings, in which like reference characters designate the same or similar parts, throughout the figures of which:
- figures 1 and 2 show perspective front and rear views of an optical apparatus with function of corneal topography apparatus according to the invention;
- figures 3 and 4 show respectively a cross sectional view and an elevational front diagrammatical view of a measuring head of said apparatus;
- figure 5 shows diagrammatically a scotopic image of a pupil detected with an apparatus according to the invention;
- figure 6 shows a photopic image of the pupil subsequent to that of figure 5;
- figure 7 shows a cross section of an exemplary embodiment alternative to that of figure 3;
- figures 8 and 9 show an apparatus according to an aspect of the invention, having a plurality of LEDs that emit a spectrum of light suitable for exciting an amount of fluoresceine put on the surface of the cornea to carry out an analysis of correct fitting of a contact lens on a cornea;
- figures 10, 11 and 12 show a preferred exemplary embodiment of an apparatus according to the invention having a plurality of visible LEDs (for example white) associated to respective filters suitable for causing only light to pass capable of exciting the fluoresceine;
- figures 13 and 14 show a further exemplary embodiment of a device according to the invention, having four infrared LEDs, two visible light LEDs and blue light LEDs, capable to carry out a topographic analysis, a pupillometric analysis and a real analysis by fluoresceine;
- figures 15 and 16 show an exemplary embodiment of a corneal topography apparatus comprising two blue LEDs for carrying out a real analysis by fluoresceine.

### Description of a preferred exemplary embodiment

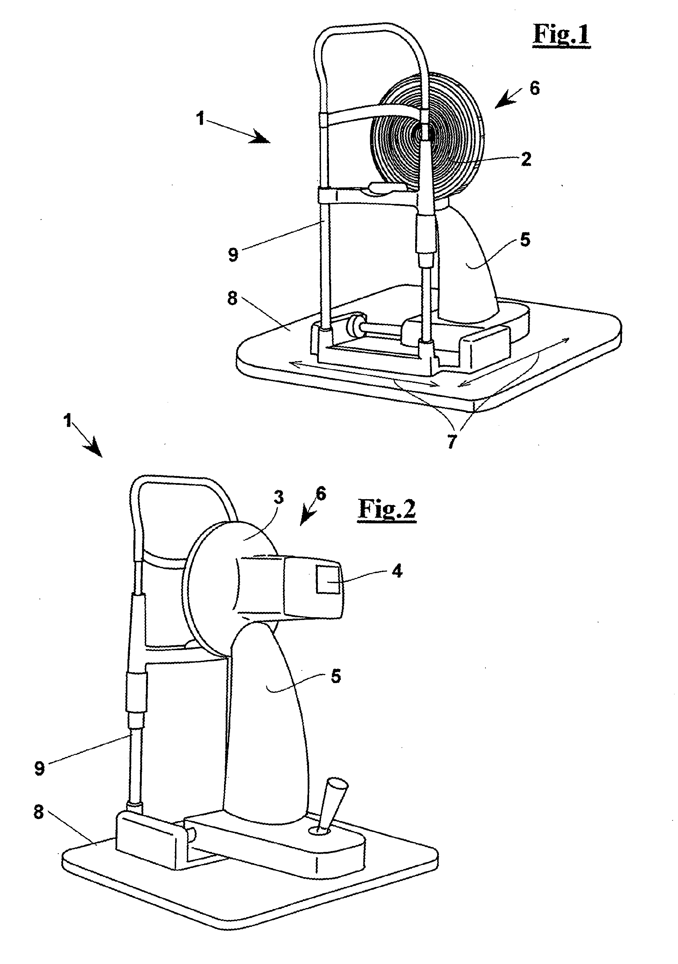

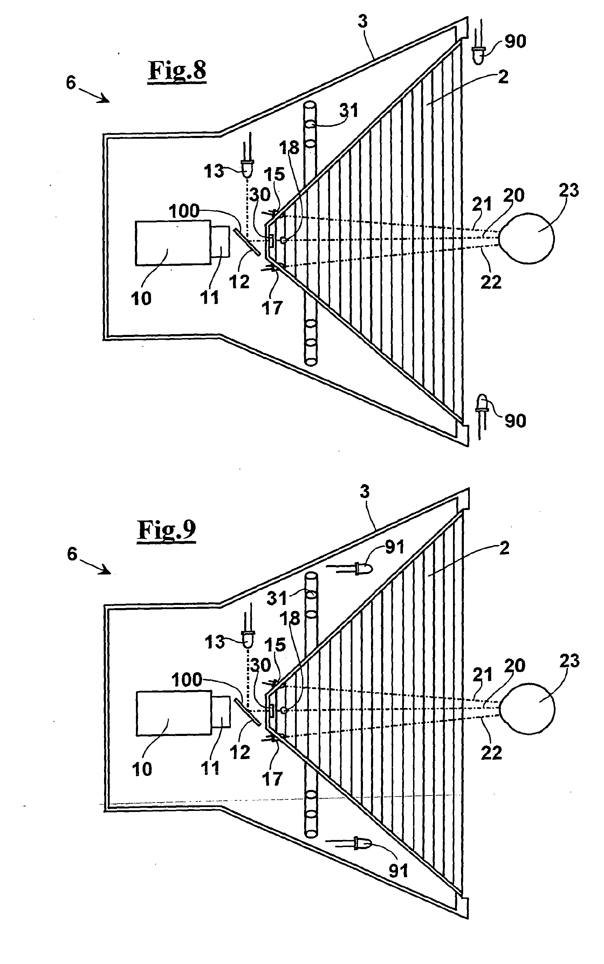

In figures 1 and 2 an elevational front view and a rear view are shown respectively of an optical apparatus with function of corneal topography apparatus 1 according to the invention, comprising a measuring head 6, connected to a support arm 5 movable along two directions 7 and 8. The device 6 comprises a Placido disc 2 that covers a carter 3 that contains an optical unit described hereinafter and has a videocamera, a lighting system and a control electronics. The image caught by the videocamera is shown on a display 4 arranged on the rear of the device 6. The corneal topography apparatus 1 rests on a fixed base frame 8 and is located in front of a support 9 for the face of a patient. This apparatus is capable of detecting images and/or films that allow to test the correct fitting of the contact lens on the eye and then to choose the contact lens that is most suitable for the geometry of an individual eye, as detailed below starting from figure 8.

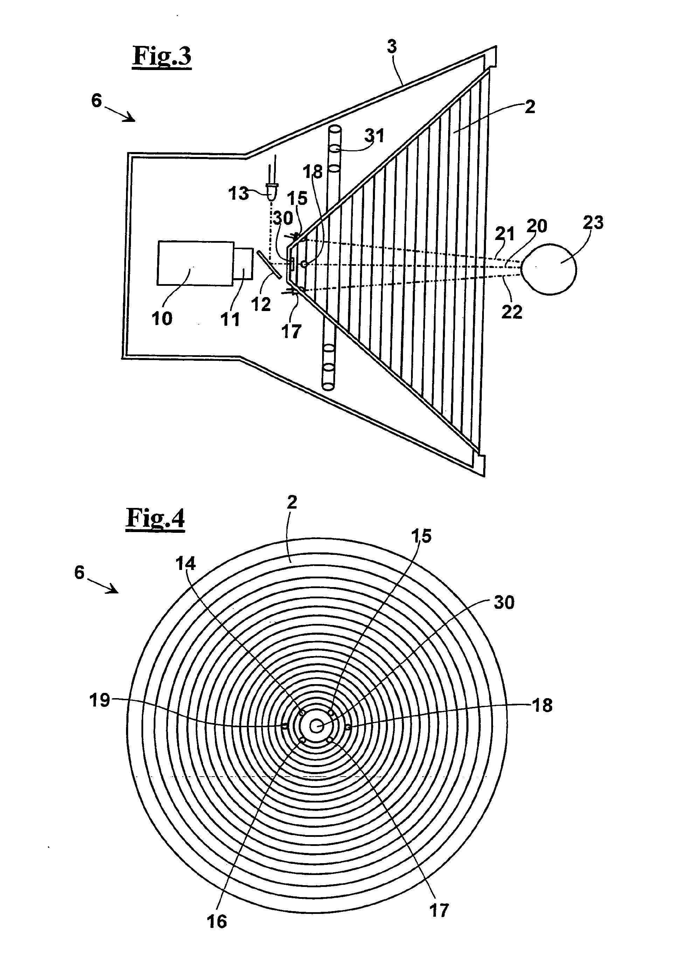

Figures 3 and 4 show respectively a cross sectional view and an elevational front view of the measuring head 6, comprising the Placido disc 2, consisting of a shell with conical shape and have a plurality of concentric opaque and transparent alternated rings, covering carter 3 that has function of support. The rear of the Placido disc 2 is backlighted by a light source 31, for example formed by a plurality of visible light LEDs, in particular red, and arranged as an arch. The light coming from LED 31, which filters through the Placido disc 2 at the transparent rings, projects on the cornea a plurality of luminous concentric rings, whose reflected light is read by a videocamera 10 and provides images (so called keratoscopic) that give information on the curvature of the cornea of the eye 23. The videocamera 10 comprises an optical unit 11 for focusing on a shot image, which is caught following an optical path that has line 20 as an axis and passes through a transparent glass or lens 30 that protects physically the rear part of the disc from powder or dirt.

The measuring head 6, according to the invention, comprises a plurality of infrared LEDs, for example respectively four LEDs 14, 15, 16, 17, arranged on the Placido disc 2 at a short distance from the centre, creating the vertices of a square concentric with disc 2 same. In particular, the above described LEDs are arranged on the smaller opaque ring of disc 2 and project light beams 21 and 22 on the eye.

Furthermore, in a preferred exemplary embodiment of the invention, the measuring head 6, described as an example, comprises two visible light LEDs 18 and 19 arranged on the Placido disc 2 at a short distance from the centre and in a position diametrically opposite to each other. In particular, such visible light LEDs 18 and 19 are arranged on the second opaque ring starting from the centre.

Preferably all the LEDs 14, 15, 16, 17, 18 and 19, which have been arranged in corresponding holes of the Placido disc, have the side surface and the base shielded with an opaque coating layer, blocking the diffusion of the light in directions that are different from a forward direction, for orienting the light same and allowing the measurements. This way, when the LEDs 14, 15, 16, 17, and/or 18 and 19 are selectively turned on, and all the other light means are turned off, the image of the disc does not reflect on the eye, giving an image clean of the same, for making for example evaluations and measurements on the pupil.

During the analysis, the patient is invited to align the eye 23 with respect to the measuring device 1, looking at a faint and hardly perceptible light point at the centre of disc 2, obtained, as well known, for reflection on a semitransparent mirror 12, so called beam splitter, inclined with respect to the path of the visible light emitted by a light source, for example a visible light LED 13.

Infrared LEDs 14, 15, 16, 17 are suitable for lighting the eye 23 with the beams 21 and 22 in a way suitable not to affect a full aperture of the pupil, since the infrared light cannot be seen by the human eye, and, at the same time, in order to allow the videocamera, which instead is sensible to infrared light, to shoot images without any visible light. This way the device 1 may have function of pupillometer and is capable to carry out a detection of the geometry of the pupil in scotopic conditions, i.e. without visible light and at a maximum pupil dilation. Visible light LEDs 18 and 19, instead, when turned on, cause the contraction of the pupil allowing to detect the geometry of the pupil in photopic conditions, i.e. at a maximum contraction.

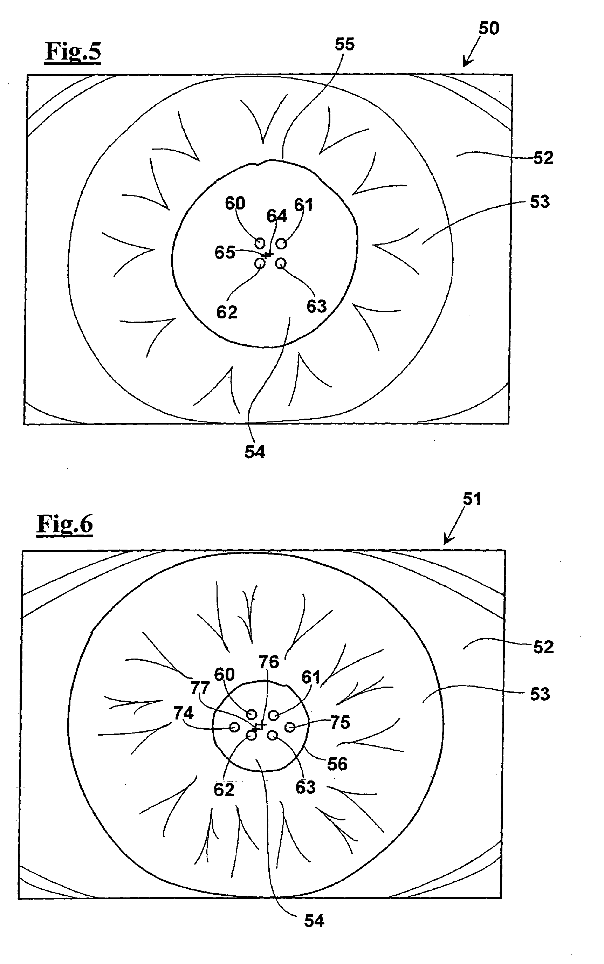

The apparatus may carry out, in addition to a static analysis, also a dynamic analysis of the pupil by means of acquisition in succession of images of the pupil changing from photopic condition to scotopic and vice-versa. Figures 5 and 6 show, in this connection, a scotopic image 50 and a photopic image 51 of a pupil, obtained with a corneal topography apparatus according to the invention.

In particular, the scotopic image 50 of figure 5, obtained without visible light, shows the pupil 54 at maximum dilation. In addition to the pupil also the iris 53 and the sclera 52 are shown. During the analysis, the infrared beams produced by the respective four LEDs 14-17 shown in figures 3 and 4, is reflected back to the cornea, thus creating, in the caught image 50, respective four luminous dots 60, 61, 62, 63, whose position depends on the curvature of the cornea same.

According to an aspect of the invention, by the calculus of the barycentre of such points the optical axis is obtained at an intersection with the cornea vertex 64. This measurement of the optical axis is of considerable precision, much higher than in the prior art. Furthermore, the same light means 14-17 are employed that are used for lighting the eye and the pupil in scotopic condition, with considerable advantages, thus reducing as far as possible an overcrowding of light means on the disc.

By an "edge detection" algorithm of known type, a control unit connected to the pupillometer according to the invention calculates the contour 55 of the pupil 54, lighted with the LEDs 14-17 of figure 3 and 4, calculating then the centre of the pupil 54, indicated by point 65.

Similarly, figure 6 shows a photopic image 51 of the eye, obtained causing the pupil 54 to contract for presence of visible light, emitted by LEDs 18 and 19 shown in figures 3 and 4. This visible light reflects on the cornea creating luminous dots 74 and 75. Even in this case the intersection of the optical axis with the cornea is calculated as the barycentre 76 of luminous dots 60, 61, 62 and 63, whereas the contour 56 of pupil 54 is determined by an "edge detection" algorithm like for the case described in figure 5.

The apparatus according to the invention is, furthermore, capable to carry out also a dynamic analysis during a transient between the extreme pupil conditions shown in figures 5 and 6.

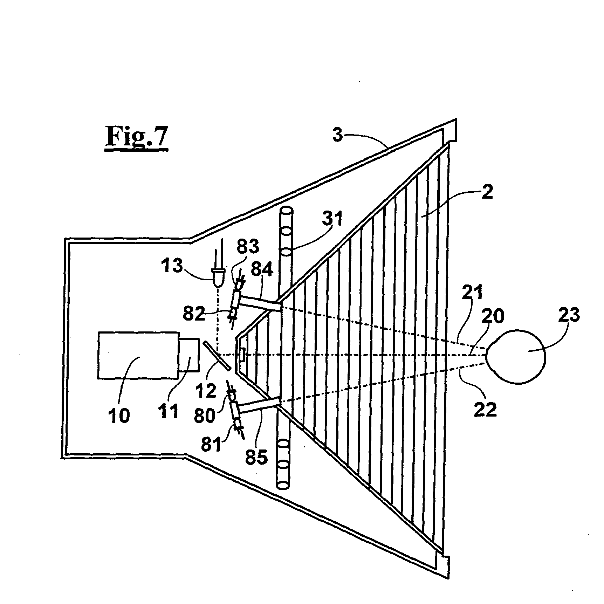

With reference now to figure 7, in an alternative exemplary embodiment of an apparatus according to the invention, a plurality is provided of light conductors, for example four, of which in the figure only the conductors 84 and 85 are shown, arranged about a central zone of disc 2 and oriented towards the eye 23. In particular, said light conductors can commute themselves between infrared light emitted by infrared LEDs, for example 83 and 81, and visible light LEDs, for example 80 and 82. Such LEDs and conductors are shielded for not giving backlight to disc 2.

Figure 8 shows an apparatus according to the invention, comprising a plurality of light sources 90, for example blue light LEDs having a peak wavelength set between 450 and 490 nm, arranged along the boundary of Placido disc 2. Such light sources have the object of exciting an amount of fluoresceine arranged between the cornea and a contact lens (not shown) applied on the cornea same of the eye 23 of the patient before the analysis. This fluoresceine, as well known, when excited by the above described blue light, emits visible light in a band, for example set between 530 and 570 nm, read by the videocamera 10. This way, the apparatus is capable of detecting images and/or films that allow to test the correct fitting of the contact lens on the eye and then to choose the contact lens that is most suitable for the geometry of an individual eye.

In particular, the excited fluoresceine produces a brighter area where it is thicker, thus showing, with varied colour tones, the different thicknesses of the gap present between lens and cornea.

This exemplary embodiment comprises also a plurality of infrared LEDs, for example respectively four LEDs 14, 15, 16, 17, arranged on the Placido disc 2 and a plurality of visible light LEDs, for example two LEDs 18 and 19 as described in all figures previous for analysis pupil.

As shown in figure 8, for a real analysis by fluoresceine on applied contact lenses a selective filter 100 can be used that lets the transmission frequencies of the fluoresceine to pass and cuts the other components, in particular the blue frequencies emitted by the excitation light source, which otherwise would add to the image, thus altering the features that are relevant for the analysis. This filter is arranged along optical path 20 between the eye and the videocamera.

The filter 100 can be arranged on beam splitter 12. In this case, the filter 100, being fixed, has to be designed in order to block the blue light that is used for exciting fluoresceine, leaving instead the wavelengths to pass of the other light sources that are used for other examinations, such as the light emitted by red LEDs 31 for backlighting the disc and infrared LEDs 14-17. In particular, the filter 100 can be for example yellow, to cut wavelength components less than 530 nm.

Alternatively, in a way not shown, the yellow filter can be removable. In this case a selective filter can be used that lets the sole wavelength of fluoresceine to pass, for example a range set between 530 and 570 nm.

Figure 9 shows a different exemplary embodiment of an apparatus according to the invention, wherein a plurality of blue light LEDs 91 is arranged behind disc 2 in order to backlight the disc same and to obtain a more diffuse light more.

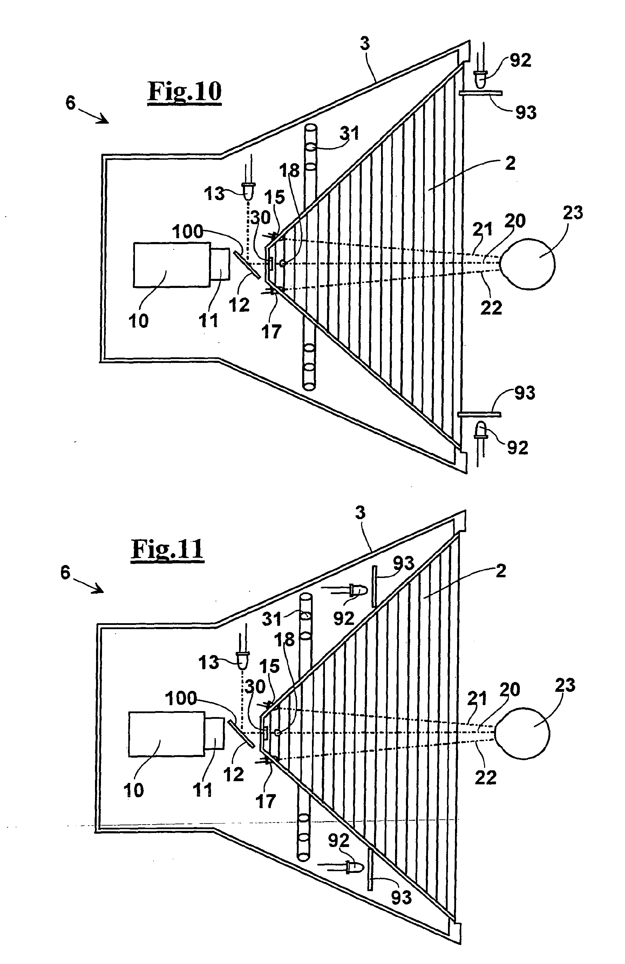

Figures 10, 11 and 12 show respectively three further exemplary embodiments of the apparatus according to the invention, having a plurality of light sources 92 with normal light and respective filters 93, which cause light capable of exciting the above described fluoresceine to pass, arranged on the cornea of the patient.

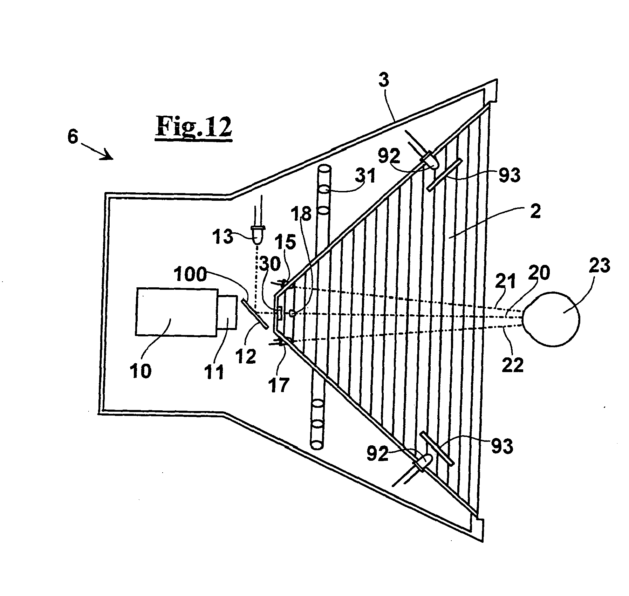

In particular: figure 10 shows an apparatus, like that described in figures 1 - 7, having a plurality of LEDs 92 with filters 93 arranged peripherally to the Placido disc 2; figure 11 shows the above described LED 92 with filter 93 arranged behind the Placido disc 2, suitable for backlighting the disc same; and figure 12 shows a further exemplary embodiment comprising a plurality of LEDs 92 with respective filter 93 applied in special holes made through the Placido disc 2.

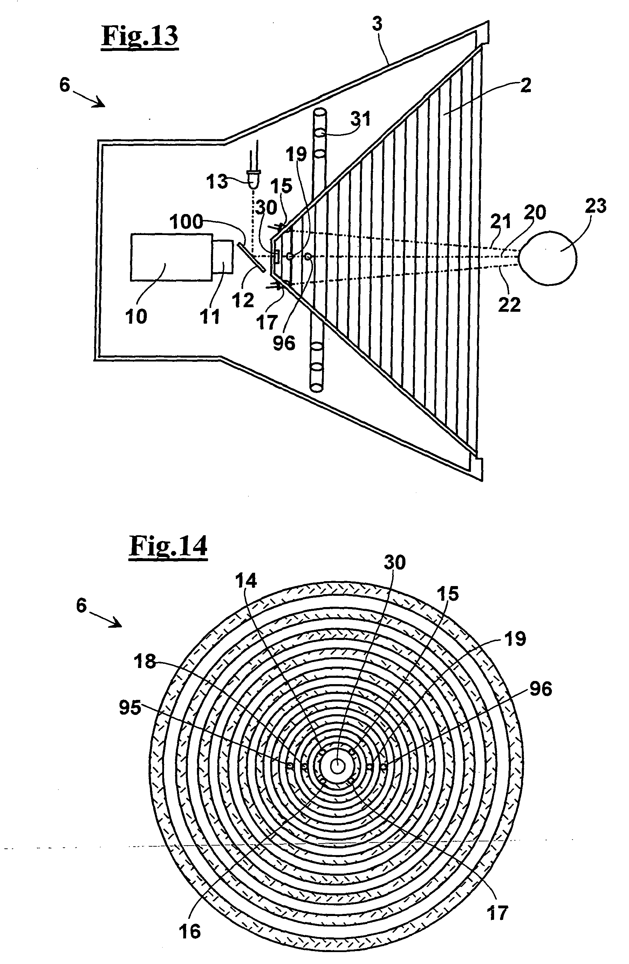

Figures 13 and 14 show a further exemplary embodiment of a device with function of corneal topography according to the invention capable to carry out a topographic analysis, a pupillometric analysis and a real analysis by fluoresceine, having two LEDs 95 and 96 that emit light in the spectrum of excitation of fluoresceine, applied near the centre of the Placido disc 2, in particular on the third opaque ring starting from the centre.

The apparatus comprises also LEDs 14, 15, 16, 17, 18, 19 that have the same functions of those described in figures 3 and 4.

Furthermore, the apparatus uses a beam splitter 12 having on a surface a fixed filter 100 capable of cutting the wavelengths that are lower than a predetermined value (for example 530 nm), suitable for causing the infrared and the transmission wavelengths of fluoresceine to pass and for cutting the light of excitation of fluoresceine that would affect a correct reading.

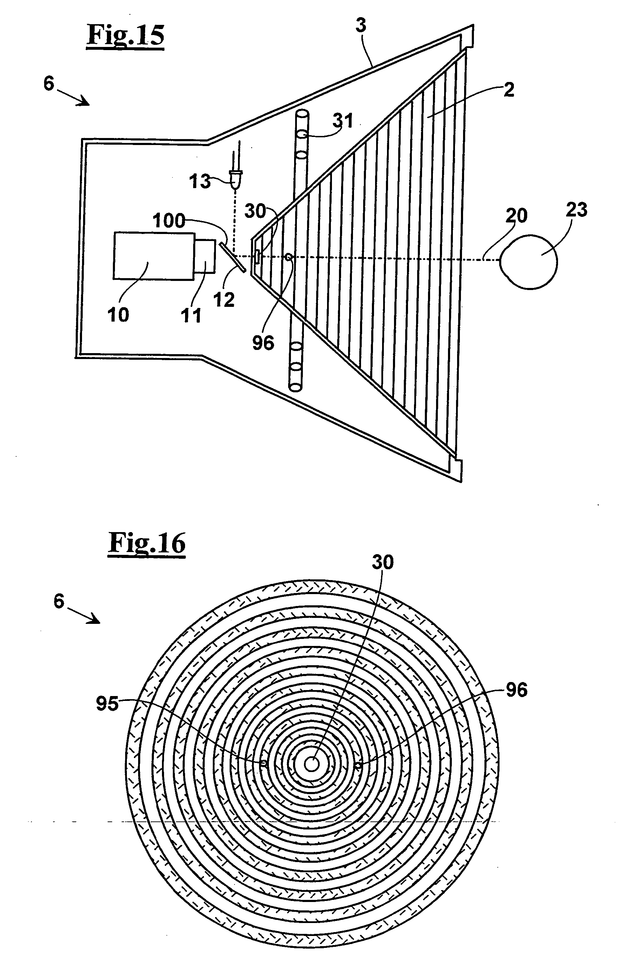

Figures 15 and 16 show another exemplary embodiment of the invention, capable to carry out a topographic analysis and an analysis by fluoresceine, but not a pupillometric analysis, since the above described apparatus comprises blue LEDs 95 and 96 and not the described infrared LEDs for pupillometry. Obviously, also this exemplary embodiment can comprise a filter 100 as described in the previous figures.

Alternatively to what shown in figures from 8 to 16, the apparatus can use a light source belonging to the spectrum of excitation of fluoresceine, oriented towards the above described beam splitter for being reflected and projected towards the eye along the optical path between the videocamera and the eye.

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

## Claims

1. A diagnostic apparatus in the field of ophthalmology comprising:
- a videocamera;
- corneal topography means associated to said videocamera, for carrying out a corneal topography of an eye,
- testing means, associated to said videocamera and to said means for carrying out a corneal topography, for testing operatively the correct fitting of said eye with a contact lens on said cornea.

2. Apparatus, according to claim 1, comprising:
- a Placido disc having a plurality of concentric alternated transparent and not transparent rings;
- means for backlighting said disc.

3. Apparatus, according to claim 1, wherein said testing means comprises:
- light means for exciting an amount of a contrast means, in particular, fluoresceine, operatively put between the cornea and the inner surface of a contact lens applied on said cornea, said videocamera being suitable for measuring the light emitted by said contrast means when excited;
- means for analysing said light emitted by said contrast means and detected by said videocamera.

4. Apparatus, according to claim 3, wherein said light means for exciting said contrast means are selected from the group comprised of:
- at least one light source, in particular a LED, which emits a bright spectrum suitable for exciting said contrast means;
- at least one light source, in particular a LED, which emits visible light, associated to a filter having a pass band containing the spectrum of excitation of said contrast means.

5. Apparatus, according to claim 4, wherein said at least one source that emits a bright spectrum suitable for exciting said contrast means is located in a position selected from the group comprised of:
- laterally along the external boundary of said Placido disc;
- back behind the Placido disc;
- applied in a respective hole made on said Placido disc;
- in a position suitable for being projected towards the eye along the fixation axis, in particular, being incident on a beam splitter.

6. Apparatus, according to claim 4, wherein said at least one source that emits a bright spectrum suitable for exciting said fluoresceine has a colour having at least one peak transmission value with wavelength suitable for exciting said fluoresceine.

7. Apparatus, according to claim 4, wherein a filter is provided selected from the group comprised of:
- a fixed filter arranged on an optical path between an eye and said videocamera, said filter being suitable for cutting wavelengths lower than a predetermined value less than a minimum wavelength of interest for analysis;
- a releasable selective filter suitable for causing only the wavelength to pass that is set in the spectrum of transmission of said contrast means, where in particular said releasable filter causes only the wavelength to pass that corresponds to the spectrum of transmission of excited fluoresceine.

8. Apparatus, according to claim 7, wherein said fixed filter is obtained on said beam splitter arranged along the optical path between the eye of the patient and the videocamera, said beam splitter being capable of cutting the wavelength lower than a predetermined value less than the minimum wavelength of interest for analysis.

9. Apparatus, according to claim 1, wherein said apparatus comprises means for determining a simulation of the position of a contact lens on the cornea, comprising:
- means for loading from a first memory the coordinates of a lens;
- means for loading from a second memory the coordinates of the cornea calculated in a previous step;
- means for subtracting the coordinates of the lens and of the cornea, obtaining data that describe a gap between lens and cornea;
- means for tracing a map of said gap on a monitor.

10. Apparatus, according to claim 1, wherein means are provided for comparing the results of the application of said testing means relative to a previous analysis with the results of the application of said testing means relative to a successive analysis.

11. Apparatus, according to claim 1, wherein said apparatus comprises:
- first light means for lighting the pupil with infrared light in a step of measuring the pupil contour;
- second light means for determining the optical axis, said second light means being coincident with said first light means;
- light means for exciting an amount of fluoresceine operatively put between the cornea and the inner surface of a contact lens applied on said cornea,
wherein in particular said first and second light means coincide with each other.

12. Apparatus, according to claim 1, wherein the following are provided:
- - means for loading from a first memory the coordinates of a lens;
- - means for loading from a second memory the coordinates of the cornea calculated in a previous step;
- - means for subtracting the coordinates of the lens and of the cornea, obtaining data that describe a gap between lens and cornea;
- - means for tracing a map of said gap on a monitor, such that a simulation can be made of the position of a contact lens on the cornea, by using.

13. Apparatus, according to claim 1, wherein said first light means and said second light means that are coincident to each other comprise a plurality of infrared light sources, in particular LEDs, located eccentrically on the surface of said Placido disc according to a geometry such that the barycentre of their images reflected on the eye coincides with said optical axis.

14. Apparatus, according to claim 1, wherein said apparatus comprises fixation light means, suitable to align an eye with said videocamera, wherein said videocamera has an optical path, said fixation light means comprising a visible fixation light source arranged laterally with respect to said optical path, said fixation light being incident on a semitransparent lamina, of beam splitter type, arranged inclined with respect to said fixation light and to said optical path, in order to direct said fixation light along said optical path. This way, said light is partially reflected by the beam splitter and crosses the centre of the Placido disc in order to be visible by the patient only when the eye is aligned with the videocamera same. Then, to align the eye the patient has simply to look at the above described fixation light source.

15. Apparatus, according to claim 13, wherein In particular, said means for backlighting said disc comprises a light source selected from the group comprised of:
- at least one fluorescent light source, in particular neon light;
- at least one infrared light source;
- at least one visible LED light source;
- a plurality of visible light LEDs, said LEDs being arranged on a circumference that is concentric to said disc and is located behind said disc.
